# EUROPEAN PATENT APPLICATION

(11) **EP 3 603 614 A1**
(43) Date of publication of application: **05.02.2020**
(21) Application number: 18775646.5
(22) Date of filing: 30.03.2018
(51) Int. Cl.: A61K 8/88, A61K 8/31, A61K 8/37, A61K 8/92, A61Q 1/02, A61Q 1/04, A61Q 1/10

(54) **SOLID PREMIXED COSMETIC PREPARATION**

(30) Priority: 30.03.2017 JP 2017068516
(71) Applicant: Kokyu Alcohol Kogyo Co., Ltd., Narita-shi Chiba 287-0225 (JP)
(72) Inventor: MASUNO, Mari, Narita-shi Chiba 287-0225 (JP); FURUHATA, Ayame, Narita-shi Chiba 287-0225 (JP)
(74) Representative: Cabinet Nuss
(86) International application number: PCT/JP2018/013665
(87) International publication number: WO 2018/181915

(57) **Abstract**

Provided is a solid premixed cosmetic preparation which imparts excellent adhesiveness to cosmetics. The solid premixed cosmetic preparation comprises: a compound of formula (I) and/or a compound of formula (II); and an oil.

## Description

### [Technical Field]

The present invention relates to a solid premixed cosmetic material comprising a specific polyamide resin.

### [Background Art]

Conventionally, various gelling agents exist as cosmetic ingredients used in the production of a cosmetic product, and polyamide resins are known as one of them. For example, as a gelling agent, an ester-terminated poly (ester-amide) (Patent literature 1), and a hydrocarbon-terminated polyether-polyamide block copolymer (Patent literature 2) have been reported. In addition, as a cosmetic product with suppressed sweating, a cosmetic product comprising an ester-terminated polyamide resin and/or an amide-terminated polyamide resin, and an amino acid gelling agent are known (Patent literatures 3 and 4).

A transparent oily liquid thickener containing polyamide-8, which is a polyamide resin, and a specific higher fatty acid or higher alcohol has been proposed (Patent literature 5).

### [Prior Art Literatures]

### [Patent Literatures]

[Patent literature 1] JP 2004-532324 A
[Patent literature 2] JP 2005-524745 A
[Patent literature 3] WO2009/139092 A1
[Patent literature 4] WO2009/139094 A1
[Patent literature 5] JP 2016-210711 A

### [Summary of the Invention]

### [Problems to Be Solved by the Invention]

Conventional cosmetic product using an ester-terminated polyamide resin and/or an amide-terminated polyamide resin is excellent in terms of transparency and suppression of sweating, etc., but the feeling of use such as adhesiveness and slipperiness of the cosmetic product using it is not necessarily enough. For example, in the case of a lipstick or lip gloss formulation, there is a problem that it is difficult to feel a lip rouge on the lip due to weak adhesiveness. There is a problem that a weakly adhesive cosmetic product does not provide a feeling of film on the lip and provides dry feeling, and it is needed to be applied many times.

In a cosmetic product using a conventional ester-terminated polyamide resin and/or an amide-terminated polyamide resin, in order to improve amount adhered, the present inventors have considered providing a soft composition with reduced hardness by a method of lowering the blending amount of the polyamide resin(s) or by blending an oil agent which is difficult to be solidified; however, in these cases, the shape retention property such as a breaking strength is deteriorated, and therefore, it is not suitable for producing a cosmetic product in the form of stick. Further, in case of a product in the form of gel such as a lip gloss, when the composition is made soft, there are also problems such as significant sweating (deterioration in storage stability) and dripping after application (dripping of gloss from the lip, and difficulty of maintaining the gloss on the lip), etc. On the other hand, if the composition is too hardened so as to maintain the shape retention property of the cosmetic product, there is a problem that the adhesiveness and slipperiness deteriorate, and it is difficult to produce a cosmetic product with a well-balanced feeling of use and the shape retention property.

Therefore, in a formulation, such as a lipstick formulation, in which a polyamide resin is blended, there is a need for a cosmetic product having excellent adhesiveness and slipperiness leading the feeling of use, in addition to the shape retention property which is basically required.

In view of the above problems, the present inventors have worked on researches on the object of providing a cosmetic material imparting a good feeling of use, particularly adhesiveness, to the final cosmetic product formulation, and a cosmetic material which also provides an excellent shape retention property in addition to the feeling of use.

### [Means for Solving the Problems]

During diligent researches to solve the above-mentioned problems, the present inventors have found that certain polyamide resins have the effect of imparting and improving the feeling of use of a gelled cosmetic product, in particular, the adhesiveness, and as a result of further studies, the present inventors have completed the present invention.
[1] A solid premixed cosmetic material, comprising a compound of formula (I) 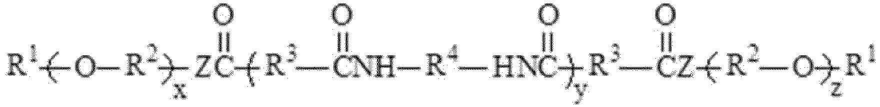 wherein, independently at each occurrence,
   R¹ is a C1-22 hydrocarbon radical;
   R² is a C2-6 hydrocarbon radical;
   R³ is a C2-52 hydrocarbon radical and, in at least one occurrence, is a 1,4-cyclohexyl diradical;
   R⁴ is selected from C2-36 hydrocarbon diradicals and C4-C100 polyether diradicals;
   Z is selected from O and NH;
   x and z are independently integers from 2 to 100; and
   y is an integer from 1 to 10, and/or
   a compound of formula (II) 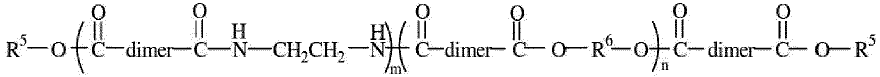 wherein, independently at each occurrence,
   R⁵ is a C16-22 hydrocarbon radical;
   R⁶ is a C2-6 hydrocarbon radical; and
   m and n are independently integers from 1 to 10; and an oil agent.
[2] The solid premixed cosmetic material according to [1], wherein the oil agent is an ester oil.
[3] The solid premixed cosmetic material according to [1] or [2] which is transparent.
[4] The solid premixed cosmetic material according to any one of [1] to [3] comprising the compound of formula (I) and the compound of formula (II).
[5] The solid premixed cosmetic material according to any one of [1] to [4] consisting of the compound of formula (I) and/or the compound of formula (II), and the oil agent.
[6] The solid premixed cosmetic material according to any one of [1] to [4] further comprising an amino acid based gelling agent.
[7] The solid premixed cosmetic material according to any one of [1] to [6], wherein the oil agent is an oil agent having a hydroxyl group.
[8] The solid premixed cosmetic material according to any one of [1] to [7] which is for a cosmetic product in the form of stick.
[9] The solid premixed cosmetic material according to any one of [1] to [8] which is for a transparent cosmetic product.

### [Advantageous Effects of the Invention]

By employing the compound of formula (I) and/or the compound of formula (II), it has become possible to provide a cosmetic product excellent in terms of the feeling of use such as adhesiveness.

Furthermore, by employing the compound of formula (I) and/or the compound of formula (II), it has become possible to provide a solid form cosmetic product which is excellent in terms of the shape retention property (such as a breaking strength) in addition to the feeling of use.

In particular, by combining the compound of formula (I) and/or the compound of formula (II) with an oil agent to form a solid premixed cosmetic material, it has become possible to provide a cosmetic material suitable for producing a cosmetic product having excellent adhesiveness.

### [Embodiments for Carrying Out the Invention]

### <The compound of formula (I)>

The compound of formula (I) is disclosed in JP 2005-524745 A, and can be produced in accordance with the production method disclosed therein. Further, the compounds of formula (I) are available under the product names: OleoCraft™ MP-32, HP-31, MP-30 etc. from Croda International Plc, United Kingdom.

### <The compound of formula (II)>

A resin composition comprising the compound of formula (II) is disclosed in JP 2004-532324 A, and can be produced in accordance with the production method disclosed therein. Further, the compound of formula (II) is available under the product name of OleoCraft™ LP-20 from Croda International Plc, United Kingdom.

### <An oil agent>

In the present invention, an "oil agent" is not particularly limited as long as it is an oil agent used in a cosmetic product, and includes an animal and vegetable fat and oil, a hydrocarbon oil, a higher fatty acid, a higher alcohol, an ester oil and the like; one or more of these oil agents may be used in combination.

In the present invention, the oil agent is preferably selected from a higher alcohol and an ester oil from the viewpoint of making a premix for a makeup cosmetic product.

Examples of the animal and vegetable fat and oil, and hydrogenated animal and vegetable fat and oil include avocado oil, perilla oil, olive oil, cacao butter, kaya oil, apricot kernel oil, hardened oil, wheat germ oil, sesame oil, rice germ oil, rice bran oil, sugarcane wax, sasanqua oil, safflower oil, shea butter, tung oil, cinnamon oil, soybean oil, tea seed oil, Camellia japonica seed oil, evening primrose oil, corn oil, rapeseed oil, germ oil, palm oil, palm kernel oil, castor oil, hardened castor oil, sunflower seed oil, grape seed oil, jojoba oil, meadowfoam oil, Argania spinosa kernel oil, macadamia nut oil, beeswax, cottonseed oil, cotton wax, Japan wax, montan wax, coconut oil, hardened coconut oil, peanut oil, lanolin, liquid lanolin, reduced lanolin, lanolin alcohol, hard lanolin, lanolin acetate, lanolin fatty acid isopropyl ester, hexyl laurate and the like.

Examples of the hydrocarbon oil include ozokerite, squalane, squalene, ceresin, paraffin, paraffin wax, liquid paraffin, pristane, polyisobutylene, polyisobutene, hydrogenated polyisobutene, microcrystalline wax, polyethylene wax, Vaseline and the like.

Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid and the like.

Examples of the ester oil include, as a monoester, isononanoic acid ester such as isononyl isononanoate, isotridecyl isononanoate, etc.; 2-ethylhexanoic acid ester such as cetyl ethylhexanoate, hexyldecyl ethylhexanoate, etc.; myristic acid ester such as isopropyl myristate, isocetyl myristate, octyldodecyl myristate, etc.; isostearic acid ester such as ethyl isostearate, isopropyl isostearate, hexyldecyl isostearate, isostearyl isostearate, cholesteryl isostearate, phytosteryl isostearate, etc.; lactic acid ester such as isostearyl lactate, octyldodecyl lactate, etc.; hydroxystearic acid ester such as ethyl hydroxystearate, ethylhexyl hydroxystearate, octyl hydroxystearate, phytosteryl hydroxystearate, cholesteryl hydroxystearate, etc.; oleic acid ester such as oleyl oleate, phytosteryl oleate, octyldodecyl oleate, etc.; neopentanoic acid ester such as isodecyl neopentanoate, isostearyl neopentanoate, tricyclodecanemethyl neopentanoate etc.; palmitate ester such as isopropyl palmitate, ethylhexyl palmitate, etc.; and others such as octyldodecyl ricinoleate, oleyl erucate, octyldodecyl erucate, lauroyl sarcosine isopropyl, and the like.

Examples of diester include diisobutyl adipate, diisopropyl adipate, diethylhexyl succinate, neopentyl glycol diisononanoate, neopentyl glycol diethylhexanoate, neopentyl glycol dicaprate, diisostearyl malate, diisopropyl dilinoleate, ethylene glycol dioctanoate, octyldodecyl stearoyloxystearate, diisopropyl sebacate, cholesteryl/octyldodecyl lauroyl glutamate, phytosteryl/octyldodecyl lauroyl glutamate, and the like.

Examples of triester include triethylhexanoin, trimethylolpropane triethylhexanoate, caprylic/capric triglyceride, triisostearin, trimethylolpropane triisostearate, erythrityl triethylhexanoate, and the like.

Examples of tetraester include pentaerythrityl tetraethylhexanoate, pentaerythrityl tetraisostearate, sucrose tetraisostearate, and the like.

Examples of polyester include polyglycerin fatty acid ester such as polyglyceryl-2 isostearate, polyglyceryl-2 diisostearate, polyglyceryl-2 triisostearate, polyglyceryl-2 tetraisostearate, and the like.

Examples of highly viscous ester oil agents include dipentaerythrityl hexahydroxystearate/hexastearate/hexarosinate, isostearic acid hydrogenated castor oil, dimer dilinoleic acid hydrogenated castor oil, polyglyceryl-2 isostearate/dimer dilinoleate copolymer, phytosteryl/isostearyl/cetyl/stearyl/behenyl dimer dilinoleate, bis-behenyl/isostearyl/phytosteryl dimer dilinoleyl dimer dilinoleate, phytosteryl isostearyl dimer dilinoleate, dimer dilinoleyl hydrogenated rosinate, dimer dilinoleyl diisostearate, dimer dilinoleyl dimer dilinoleate, cholesteryl/behenyl/octyldodecyl lauroyl glutamate, phytosteryl/behenyl/octyldodecyl lauroyl glutamate, phytosteryl/decyltetradecyl myristoyl methyl alaninate, diglycerin/dilinoleic acid/hydroxystearic acid copolymer, and the like.

Examples of higher alcohols include lauryl alcohol, myristyl alcohol, stearyl alcohol, eicosanol, behenyl alcohol, oleyl alcohol, hexyldecanol, isostearyl alcohol, octyldodecanol, decyltetradecanol, dodecylhexadecanol, and the like.

In one embodiment of the present invention, from the viewpoint of gelation by a hydrogen bond, the oil agent is preferably an oil agent having a hydroxyl group such as diisostearyl malate, ethyl hydroxystearate, polyglyceryl-2 triisostearate, sucrose tetraisostearate, and the like; and diisostearyl malate is particularly preferred.

In one embodiment of the present invention, from the viewpoint of making a premix for a transparent cosmetic product, it is also preferred that the oil agents further comprise isononanoic ester, in particular isotridecyl isononanoate.

### <Amino acid based gelling agent>

In the present invention, an "amino acid based gelling agent" is a gelling agent having amino acid residue.

By using the amino acid based gelling agents in combination, a highly transparent cosmetic material and cosmetic product can be provided.

In general, a cosmetic product using the amino acid based gelling agent tends to have poor adhesiveness, and the effect exhibited by employing the solid premixed cosmetic material of the present invention is significant.

Specifically, the amino acid derivatives represented by the following formula (III) are exemplified: 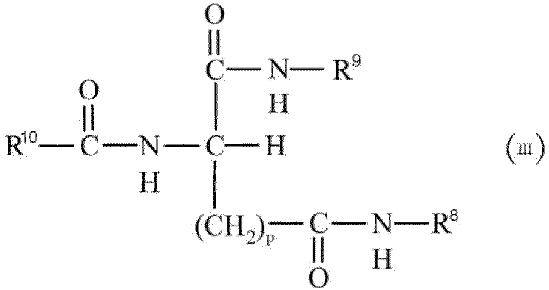 wherein
R⁸ and R⁹ are a C1 to C10 linear or branched alkyl group which may be the same as or different from each other;
R¹⁰ is a C1 to C26 linear or branched alkyl group; p is an integer of 1 or 2.

From the viewpoints of
(a) Gelation of an oil agent, stabilization of emulsified system (Functions related to stability),
(b) Improvement of the feel, and adjustment of the spinnability when formulated into cosmetic materials (Functions related to sensory), and
(c) Cosmetic material with high transparency can be easily obtained (Functions related to visual), etc.,
   as the amino acid oil based gelling agents, dibutyl ethylhexanoyl glutamide and dibutyl lauroyl glutamide are particularly preferred, and specific product names include EB-21 and GP-1 (both manufactured by Ajinomoto Co., Inc.). Since dibutyl ethylhexanoyl glutamide (EB-21) has a strong bitter taste, it is preferable to use dibutyl lauroyl glutamide (GP-1), and it is most preferred to use the combination of two of the above-mentioned dibutyl ethylhexanoyl glutamide and dibutyl lauroyl glutamide.

### <Solid premixed cosmetic material>

In the present specification, "a solid premixed cosmetic material" is a solid form mixture comprising a compound of formula (I) and/or a compound of formula (II), and an oil agent, and is a raw material employed for producing a cosmetic product.

By making a solid form premix, there are advantages such as easy transportation as a raw material; and by lowering melting point of the cosmetic material by combining an oil agent with polyamide resin, there are advantages such as safe handling, as well as easy heating/melting and thus easy blending and mixing in the production of various cosmetic products.

In one embodiment of the present invention, the solid premixed cosmetic material comprises 20 to 95 % by weight, preferably 30 to 95 % by weight, and more preferably 40 to 90 % by weight of the compound of formula (I) and/or the compound of formula (II), from the viewpoint of easy handling in the production of a cosmetic product.

In one embodiment of the present invention, the solid premixed cosmetic material comprises 5 to 80 % by weight, preferably 5 to 70 % by weight, and more preferably 10 to 60 % by weight of the oil agent, from the viewpoint of easy handling in the production of a cosmetic product.

In one embodiment of the present invention, the ratio of the compound of the formula (I) and/or the compound of the formula (II) and the oil agent, contained in the solid premixed cosmetic material can be appropriately selected, but it comprises the compound of the formula (I) and/or the compound of the formula (II) : the oil agent in the range of proportion of 2: 8 to 9.5:0.5, preferably 3:7 to 9.5:0. 5, more preferably in the ratio of 4:6 to 9:1, from the viewpoint of easy handling such as easy mixing and melting in the production of a cosmetic product.

In one embodiment of the present invention, the solid premixed cosmetic material consists only of the compound of the formula (I) and/or the formula (II) and the oil agent, from the viewpoint of enabling application to various cosmetic products.

In another embodiment of the present invention, the solid premixed cosmetic material comprises other cosmetic ingredients; examples of other cosmetic ingredients include, but not limited to, an alcohol, water, a gelling agent, a thickener, a surfactant, an antioxidant, a preservative, and the like. Further, it may comprise a pH modifier, a chelating agent, a plant extract, a warming/cooling ingredient (warming: Capsicum Frutescens Fruit Extract, etc., cooling: menthol etc.), an inorganic colorant, an organic colorant, and the like.

In one embodiment of the present invention, the solid premixed cosmetic material does not comprise an ester-terminated polyamide resin such as Sylvaclear A200V and an amide-terminated polyamide resin, from the viewpoint of providing sufficient adhesiveness to a cosmetic product to be produced and the viewpoint of making a highly transparent cosmetic material.

The solid premixed cosmetic material can be produced by a known production method of a cosmetic material mixture. For example, it can be obtained by dissolving each cosmetic ingredient with stirring to obtain a uniform mixture thereof.

In one embodiment of the present invention, the solid premixed cosmetic material is a solid form, from the viewpoint of easy handling, especially avoiding problems such as leakage during transportation.

In one embodiment of the present invention, the solid premixed cosmetic material is transparent, from the viewpoint of versatility. In a preferred embodiment of the present invention, the solid premixed cosmetic material is transparent, and does not comprise a colorant etc.

In one embodiment of the present invention, the solid premixed cosmetic material comprises the compound of formula (I) and the compound of formula (II), and their ratio varies depending on the final cosmetic product formulation, but typically, it comprises the compound of formula (I) and the compound of formula (II) in the proportion of 1:9 to 9:1, preferably 2:8 to 8:2.

Since the effect of providing and improving adhesiveness is high in a cosmetic product comprising an amino acid based gelling agent, the present inventive solid premixed cosmetic material may further comprise an amino acid based gelling agent.

In one embodiment of the present invention, the solid premixed cosmetic material comprises 0.3 to 10.0 % by weight, preferably 0.5 to 5.0 % by weight, and more preferably 0.5 to 3.0 % by weight of the amino acid based gelling agent.

In one embodiment of the present invention, the solid premixed cosmetic material comprises the compound of formula (I) and/or the compound of formula (II) and the amino acid based gelling agent in the proportion of 0:10 to 10:0, preferably 1:9 to 9:1.

### <Cosmetic product>

The solid premixed cosmetic material of the present invention can be used for producing any cosmetic products. The cosmetic product is not particularly limited, but means any products applied on the skin, the hair, the lip etc. for the purpose of cleaning the body or making a beautiful look. Specific examples include a skin care product, a makeup product, a hair care product, a body care product, and the like, but are not limited thereto.

The present invention also provides a cosmetic product employing the solid premixed cosmetic material. In addition to the solid premixed cosmetic material of the present invention, the cosmetic product may comprise an oil agent, a silicone oil, an alcohol, water, a gelling agent, a thickener, a surfactant, an antioxidant, a preservative, a pH modifier, a chelating agent, a plant extract, a warming/cooling ingredient (warming: Capsicum Frutescens Fruit Extract, etc., cooling: menthol, etc.), a coloring agent such as an inorganic colorant, an organic colorant, a natural pigment, an oil-soluble dye, a water-soluble dye, a flavor/fragrance, an additive such as an UV absorbent, and an active ingredient such as a whitening ingredient and the like, which are commonly used in a cosmetic product.

Here, the oil agent used in the cosmetic product may be the same as or different from the oil agent comprised in the solid premixed cosmetic material.

The formation of the cosmetic product of the present invention is not limited. For example, it is selected from an antiperspirant, a cream, a lip gloss, an eye shadow, a mascara, a lipstick (a lip rouge), a hair gel, a hair wax, a hair stick, a hair cream, a sun care product, a foundation, an eye color, and the like, and can be made into suitable formation for each application.

In one embodiment of the present invention, since the solid premixed cosmetic material of the present invention also provides the cosmetic product with the shape retention property and the breaking strength, it is preferable to use it in a solid form cosmetic product, especially in a cosmetic product in the form of stick. Examples of a cosmetic product in the form of stick include a lip cream, a lip stick, an antiperspirant, a UV stick, a cheek color, an eyebrow, an eyeliner, a concealer, a stick foundation, a hair dressing agent and the like, but are not limited thereto.

In one embodiment of the present invention, since the solid premixed cosmetic material of the present invention can provide a highly transparent cosmetic product, it is suitable for a makeup product. Examples of a makeup cosmetic product include a makeup base, a foundation, a lip color, a lipstick, a lip cream, a lip gloss, a cheek color, an eyeliner, a mascara, an eye shadow, an eyebrow and the like, but are not limited thereto.

In one embodiment of the present invention, since the solid premixed cosmetic material of the present invention can provide a highly adhesive cosmetic product, it can provide a cosmetic product excellent in coloring when it is applied. Thus, the solid premixed cosmetic material of the present invention is suitable for a cosmetic product comprising a coloring agent.

In one embodiment of the present invention, since the solid premixed cosmetic material is highly transparent, and is colorless and transparent in a preferable embodiment, the exact color of the coloring agent employed in the cosmetic product can be came out. Form this viewpoint as well, it is suitable for a cosmetic product comprising a coloring agent.

In one embodiment of the present invention, since the solid premixed cosmetic material is highly transparent, and is colorless and transparent in a preferable embodiment, it is suitable to be used in a colorless and transparent cosmetic product which develops color when it is applied on the skin or the lip.

The cosmetic product can be produced by a known method for producing a cosmetic product. For example, the solid premixed cosmetic material of the present invention and other cosmetic ingredients are dissolved with stirring to obtain a uniform mixture thereof, and then an additive such as a flavor/fragrance is added thereto and the resulting mixture is molded to produce a cosmetic product.

In one embodiment of the present invention, a method of producing a cosmetic product comprising dissolving the solid premixed cosmetic material of the present invention, and mixing it with other cosmetic ingredients is provided.

In one embodiment of the present invention, a method of improving adhesiveness of a cosmetic product comprising dissolving the solid premixed cosmetic material of the present invention, and mixing it with other cosmetic ingredients is provided.

Hereinafter, the present invention will be described in more detail based on examples. However, the present invention is not limited to these examples, and various modifications can be made without departing from the technical idea of the present invention. In the present specification, unless otherwise specified, % means % by weight.

### [EXAMPLES]

The five grade evaluation in the examples, unless otherwise specified, is a result of relative evaluation in five grades based on the following criteria in each example.
5: Good
4: Generally good
3: Normal
2: Slightly bad
1: Bad

### [Example 1] Stick formulation 1

The properties of the gelled product in the form of stick obtained by gelling the ester oil with each polyamide resin were verified. The blending amount of the polyamide resin was adjusted so that each sample would have almost the same level as much as possible.

Breaking strength, slipperiness, adhesiveness, and storage stability (the presence of sweating) of the obtained samples were evaluated.

The compositions and the results are shown in Table 1.

### <Adhesiveness>

The sample was fixed vertically downward with a fixing jig, the sample surface was applied three times to the same part of the surface of the artificial leather at a constant speed, and the average of the amount of each sample adhered was measured. Measurement was made with the sample stage and the artificial leather kept at a temperature of 35 °C. (At the time of measurement, the load on the stick was 20g) Evaluation was conducted in five grades.

### <Hardness>

A measurement was carried out using an EZ-Test-20N hardness meter (manufactured by Shimadzu Corporation). The needle diameter was 1.0 mm∅, and the maximum value measured for the stress (N) at a needle penetration depth of 10 mm with a test speed of 10 mm/min was defined as the hardness.

### <Breaking strength>

A measurement was carried out using the hardness meter used in hardness measurement. A lip rouge container or a stick-shaped container was fixed horizontally, a load jig was attached to a side face of a wound-out stick at a position 10 mm from the end of a receiving plate of the stick (the base of the lipstick, etc.), and a load was applied under conditions of a stick temperature of 25°C and a speed of 50 mm/min. The stress (N) at breakage was measured, and the maximum value was defined as the breaking strength. The lipstick used was a stick molded using a lip rouge mold having a diameter of 11.5 mm.

### <Breaking point>

In the breaking strength test, the depth (mm) until the stick was broken was measured and used as the breaking point.

### <Sweating>

After the sample was stored at 45 °C for 30 minutes and 1 hour, respectively, the amount of sweating on the surface of the sample was judged visually. Evaluation results are displayed as "o" when the amount of sweating is small and no oil droplet is observed, "Δ" when the sweating is slightly observed, and "×" when the amount of sweating is large.

### <Shape retention property>

Lip rouge left standing for 24 hours in an incubator at 25 °C was used.

Shape retention property was checked by a monitor of 10 persons, and the touch feeling (hardness and softness) when applied to the lip was relatively evaluated based on the following criteria.
5: Good hardness
4: Generally good hardness
3: Normal
2: Soft
1: Very soft

**Table 1: Comparison of polyamide resins**

| | | Com. Ex 1 | Example 1 |
|---|---|---|---|
| Polyamide resin | Sylvaclear A200V | 15.0 | |
| | OleoCraft MP-32 | | 17.0 |
| Oil agent | HAIMALATE DIS | 85.0 | 83.0 |
| | Total | 100.0 | 100.0 |
| | | | |
| Hardness (N) | | **0.16** | **0.15** |
| Breaking strength (N) | | **0.63** | **0.72** |
| Breaking point: Stroke (mm) | | **3.05** | **4.48** |
| Amount adhered (mg) | 1^{st} Time | 10.33 | 13.35 |
| | 2^{nd} Time | 11.03 | 12.56 |
| | 3^{rd} Time | 10.36 | 10.92 |
| | Ave. | 10.57 | **12.28** |
| Sweating (Visual evaluation) | After 30 minutes | ○ | ○ |
| | After 1 hour | ○ | ○ |
| Shape retention property | | **3** | **4** |

From the above results, it can be understood that MP-32, which is a compound of the formula (I), provides excellent strength, excellent adhesiveness, and excellent shape retention property compared to a conventional polyamide resin.

Comparative Example 1 comprising Sylvaclear A200V is slightly yellowish, whereas Example 1 comprising MP-32, which is a compound of formula (I), is more transparent.

### [Example 2] Stick formulation 2

The properties of a gelled product in the form of stick obtained by gelling the ester oil with each polyamide resin were verified. The blending amount of the polyamide resin was adjusted so that each sample would have almost the same level as much as possible.

Breaking strength, adhesiveness, and shape retention property were evaluated in the same manner as in Example 1.

The compositions and the results are shown in Table 2.

**Table 2: Comparison of the combinations of polyamide resins**

| | | Ex. 2A | Ex. 2B | Ex. 2C |
|---|---|---|---|---|
| Polyamide resin | Sylvaclear A200V | 14.0 | 13.0 | |
| | OleoCraft LP-20 | 3.0 | | 23.0 |
| | OleoCraft MP-32 | | 3.0 | 5.0 |
| Oil agent | HAIMALATE DIS | 83.0 | 84.0 | 72.0 |
| | Total | 100.0 | 100.0 | 100.0 |
| | | | | |
| Hardness (N) | | **0.17** | **0.16** | **0.18** |
| Breaking strength (N) | | **0.65** | **0.81** | **1.33** |
| Breaking point: Stroke (mm) | | **3.10** | **3.24** | **4.99** |
| Amount adhered (mg) | 1^{st} Time | 14.71 | 16.38 | 17.39 |
| | 2^{nd} Time | 14.94 | 16.11 | 17.32 |
| | 3^{rd} Time | 15.26 | 13.96 | 15.71 |
| | Ave. | **14.97** | **15.48** | **16.81** |
| Shape retention property | | **3** | **4** | **5** |

From the above results, it can be understood that remarkable adhesiveness can be obtained by combining MP-32, which is a compound of formula (I), and LP-20, which is a compound of formula (II).

### [Example 3] Lip gloss formulation

The production of lip gloss having the respective composition shown in Table 3 below was carried out by a conventional method.

The adhesiveness and slipperiness of the obtained lip glosses were measured by the following method. The evaluation of the characteristics was performed in a five grade relative evaluation from the measurement results of all the samples of the examples and comparative examples. The results are shown in Table 3.

### <Adhesiveness>

Sensory evaluation of adhesiveness was carried out by 10 panelists.

Averages were calculated based on a five grade evaluation (5: very good, 4: good, 3: normal, 2: bad, 1: very bad), and then they are ranked as follows.
5: Adhesiveness is very good
   (Average of five grade evaluation is 4.0 or more)
4: Adhesiveness is good
   (Average of five grade evaluation is 3.0 or more and less than 4.0)
3: Appropriate adhesiveness
   (Average of five grade evaluation is 2.0 or more and less than 3.0)
2: Poor adhesiveness
   (Average of five grade evaluation is 1.0 or more and less than 2.0)
1: Very poor adhesiveness
   (Average of five grade evaluation is less than 1.0)

### <Slipperiness>

Sensory evaluation of slipperiness was carried out by 10 panelists.

Averages were calculated based on a five-grade evaluation (5: very good, 4: good, 3: normal, 2: bad, 1: very bad), and then they are ranked as follows.
5: Spreadability is very good
   (Average of five grade evaluation is 4.0 or more)
4: Spreadability is good
   (Average of five grade evaluation is 3.0 or more and less than 4.0)
3: Appropriate spreadability
   (Average of five grade evaluation is 2.0 or more and less than 3.0)
2: Poor spreadability
   (Average of five grade evaluation is 1.0 or more and less than 2.0)
1: Very poor spreadability
   (Average of five grade evaluation is less than 1.0)

**Table 3: Comparison of the effects of polyamide resin in lip gloss formulations**

| | Com. Ex 3 | Example 3A | Example 3B |
|---|---|---|---|
| **Sylvaclear A200V** | **3.00** | | |
| **OleoCraft LP-20** | | **3.00** | |
| **OleoCraft MP-32** | | | **3.00** |
| GP-1 | 0.07 | 0.07 | 0.07 |
| EB-21 | 0.04 | 0.04 | 0.04 |
| HAIMALATE DIS | 30.00 | 30.00 | 30.00 |
| RISONOL 20SP | 8.00 | 8.00 | 8.00 |
| KAK PTI | 8.89 | 8.89 | 8.89 |
| Hydrogenated polyisobutene | 50.00 | 50.00 | 50.00 |
| | | | |
| Adhesiveness | 4 | 5 | 5 |
| Slipperiness | 3 | 5 | 4 |

From the above results, it can be understand that it is possible to obtain a lip gloss excellent in adhesiveness and slipperiness by combining the compound of formula (I) or the compound of formula (2) with an oil agent as compared with the one using the amide-terminated polyamide resin.

### [Examples 4 to 8] Lipstick formulations

Productions of lipsticks having the respective compositions shown in Tables 4 to 8 below were carried out by a conventional method. The blending amount of the polyamide resin was adjusted so that each sample would have a hardness range suitable for a lipstick (1.5 to 2.0 N).

Adhesiveness and slipperiness were measured in the same manner as in Example 1. The results of the five grade evaluation are shown in Tables 4 to 8, respectively.

**Table 4: Comparison of the effects of polyamide resin in lipstick formulations**

| | | Com. Ex 4 | Example 4 |
|---|---|---|---|
| Polyamide resin | Sylvaclear A200V | 13.00 | |
| | OleoCraft LP-20 | | 2.00 |
| | OleoCraft MP-32 | | 11.00 |
| Amino acid based gelling agent | GP-1 | 0.90 | 0.90 |
| | EB-21 | 0.60 | 0.60 |
| Ester oil | HAIMALATE DIS | 13.00 | 13.00 |
| | KAK 139 | 52.00 | 52.00 |
| | Tricyclodecanemethyl neopentanoate | 20.50 | 20.50 |
| | | | |
| Evaluations | Shape retention property | 5 | 5 |
| | Adhesiveness | 4 | 5 |
| | Slipperiness | 4 | 5 |

**Table 5: Comparison of the effects of polyamide resin in lipstick formulations**

| | | Com. Ex 5 | Example 5 |
|---|---|---|---|
| Polyamide resin | Sylvaclear A200V | 15.00 | |
| | OleoCraft LP-20 | | 3.00 |
| | OleoCraft MP-32 | | 12.00 |
| Amino acid based gelling agent | GP-1 | 0.90 | 0.90 |
| | EB-21 | 0.60 | 0.60 |
| Ester oil | HAIMALATE DIS | 15.00 | 15.00 |
| | KAK 139 | 68.50 | 68.50 |
| | | | |
| Evaluations | Shape retention property | 5 | 5 |
| | Adhesiveness | 2 | 4 |
| | Slipperiness | 2 | 4 |

**Table 6: Comparison of effects of polyamide resin in lipstick formulations**

| | | Com. Ex 6 | Example 6 |
|---|---|---|---|
| Polyamide resin premix | SylvaclearA200V | 15.00 | |
| | OleoCraft LP-20 | | 9.00 |
| | OleoCraft MP-32 | | 9.00 |
| | HAIMALATE DIS | 15.00 | 12.00 |
| Amino acid based gelling agent | AJK-OD2046 | 15.00 | 15.00 |
| Ester oil | KAK 139 | 55.00 | 55.00 |
| Evaluations | Shape retention property | 5 | 5 |
| | Adhesiveness | 2 | 5 |
| | Slipperiness | 3 | 4 |

**Table 7: Comparison of effects of polyamide resin in lipstick formulations**

| | Com. Ex 7 | Example 7A | Example 7B | Example 7C |
|---|---|---|---|---|
| Sylvaclear A200V | 15.00 | | | |
| OleoCraft LP-20 | | 9.00 | 25.50 | |
| OleoCraft MP-32 | | 9.00 | | 14.10 |
| HAIMALATE DIS | 15.00 | 12.00 | 4.50 | 15.90 |
| AJK-OD2046 | 15.00 | 15.00 | 15.00 | 15.00 |
| KAK 139 | 55.00 | 55.00 | 55.00 | 55.00 |
| | | | | |
| Shape retention property | 5 | 5 | 4 | 4 |
| Adhesiveness | 2 | 5 | 5 | 4 |
| Slipperiness | 3 | 4 | 5 | 4 |

**Table 8: Comparison of effects of polyamide resin in lipstick formulations**

| | Com. Ex 8 | Example 8A | Example 8B | Example 8C |
|---|---|---|---|---|
| Sylvaclear A200V | 15.00 | | | |
| OleoCraft LP-20 | | 25.50 | 25.50 | |
| OleoCraft MP-32 | | | | 15.00 |
| HAIMALATE DIS | 15.00 | 4.50 | | |
| RISOREX PGIS22 | | | 4.50 | |
| KAK TCIN | | | | 15.00 |
| AJK-OD2046 | 15.00 | 15.00 | 15.00 | 15.00 |
| KAK 139 | 55.00 | 55.00 | 55.00 | 55.00 |
| | | | | |
| Shape retention property | 5 | 4 | 5 | 5 |
| Adhesiveness | 2 | 4 | 5 | 3 |
| Slipperiness | 3 | 4 | 4 | 4 |

From the above results, it can be understand that it is possible to obtain a lipstick excellent in adhesiveness and slipperiness by combining the compound of the present formula (I) or the compound of the formula (2) with an oil agent as compared with the one using the amide-terminated polyamide resin.

In particular, it can be understood that the effect of improving the adhesiveness is remarkable in the combination with the amino acid gelling agent.

Also, it can be understood that the effect of adhesiveness is obtained in combination with various oil agents.

The names of the cosmetic ingredients used in the examples and their ingredient names listed on the cosmetic products are shown below.

### Abbreviation

| [Product Name] | [INCI Name] |
|---|---|
| HAIMALATE PAM | Diisostearyl malate, Ethylenediamine / hydrogenated dimer dilinoleate copolymer bis-di-c14-18 alkyl amide |
| Sylvaclear A200V | Ethylenediamine / hydrogenated dimer dilinoleate copolymer bis-di-c14-18 alkyl amide |
| OleoCraft LP-20 | Polyamide-8 |
| OleoCraft MP-32 | Polyamide-3 |
| GP-1 | Dibutyl lauroyl glutamide |
| EB-21 | Dibutyl ethylhexanoyl glutamide |
| AJK-OD2046 | Octyldodecanol, Dibutyl lauroyl glutamide, Dibutyl ethylhexanoyl glutamide |
| HAIMALATE DIS | Diisostearyl malate |
| KAK 139 | Isotridecyl isononanoate |
| RISOREX PGIS22 | Polyglyceryl-2 diisostearate |
| KAK TCIN | Tricyclodecanemethyl isononanoate |
| RISONOL 20SP | Octyldodecanol |
| KAK PTI | Pentaerythrityl tetraisostearate |

## Claims

1. A solid premixed cosmetic material comprising a compound of formula (I) 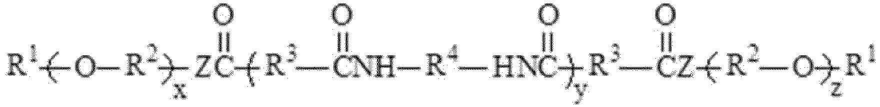 wherein, independently at each occurrence,
R¹ is a C1-22 hydrocarbon radical;
R² is a C2-6 hydrocarbon radical;
R³ is a C2-52 hydrocarbon radical and, in at least one occurrence, is a 1, 4-cyclohexyl diradical;
R⁴ is selected from C2-36 hydrocarbon diradicals and C4-C100 polyether diradicals;
Z is selected from O and NH;
x and z are independently integers from 2 to 100; and
y is an integer from 1 to 10, and/or
a compound of formula (II) 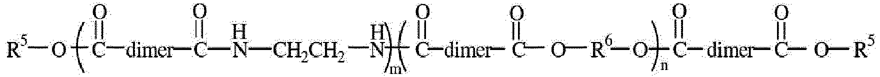 wherein, independently at each occurrence,
R⁵ is a C16-22 hydrocarbon radical;
R⁶ is a C2-6 hydrocarbon radical; and
m and n are independently integers from 1 to 10; and an oil agent.

2. The solid premixed cosmetic material according to Claim 1, wherein the oil agent is an ester oil.

3. The solid premixed cosmetic material according to Claim 1 or 2 which is transparent.

4. The solid premixed cosmetic material according to any one of Claims 1 to 3 comprising the compound of formula (I) and the compound of formula (II).

5. The solid premixed cosmetic material according to any one of Claims 1 to 4 consisting of the compound of formula (I) and/or the compound of formula (II), and the oil agent.

6. The solid premixed cosmetic material according to any one of Claims 1 to 4 further comprising an amino acid based gelling agent.

7. The solid premixed cosmetic material according to any one of Claims 1 to 6, wherein the oil agent is an oil agent having a hydroxyl group.

8. The solid premixed cosmetic material according to any one of Claims 1 to 7 which is for a cosmetic product in the form of stick.

9. The solid premixed cosmetic material according to any one of Claims 1 to 8 which is for a transparent cosmetic product.
